# EUROPEAN PATENT SPECIFICATION

(11) **EP 3 578 673 B1**
(45) Date of publication and mention of the grant of the patent: **20.03.2024**
(21) Application number: 18203528.7
(22) Date of filing: 14.12.2015
(51) Int. Cl.: C22B 3/18, C12N 1/20, C22B 15/00

(54) **TANK BIOLEACHING OF COPPER SULFIDE ORES**
TANKBIOLAUGUNG VON KUPFERSULFIDERZEN
CUVE DE BIOLIXIVIATION DE MINERAIS DE SULFURE DE CUIVRE

(30) Priority: 15.12.2014 IR 13933010240
(43) Date of publication of application: 11.12.2019
(62) Divisional of application: 15199787.1
(73) Proprietor: Middle East Mine and Industry Company, 1997744111 Tehran (IR)
(72) Inventor: Pourmand, Aliasghar, 1997744111 Tehran (IR)
(74) Representative: Hamm&Wittkopp Patentanwälte PartmbB

(56) References cited:
- WO-A1-02/066689
- US-A1- 2004 206 208
- US-A1- 2009 061 503

## Description

### FIELD OF THE INVENTION

The present invention relates to an inoculant for improved bioleaching method for metal sulfide ores by indigenous bacterial strains. In particular, the invention provides a continuous tank bioleaching method for separating copper from copper sulfide ores and in particular from concentrated copper sulfide ores.

### BACKGROUND

There is a continuously increasing global copper demand, while the most profitable copper resources have already been exhausted and the content of the metal in the remaining natural ore deposits is decreasing. This situation results in a continuous demand for new, improved and economically feasible processes of copper winning. Copper is commonly present in the earth's crust as copper-iron sulfide and copper sulfide minerals such as chalcopyrite (CuFeS₂), covellite (CuS), chalcocite (CuSz), bornite (Cu₅Fe₄), or engarite (Cu₅AsS₅). 85 % of the worldwide copper deposits are copper sulfide ores with chalcopyrite as the most common copper ore. The most important method for separating copper from its sulfide ores is pyrometallurgy. However, those methods need technically challenging equipment, are energy consuming and, due to the formation of sulfur dioxide, are harmful to the environment. Hence, an economic feasible pyrometallurgical process requires that the rocks subjected to copper separation contain a high content of copper ore. In order to separate copper from low grade copper ores, hydrometallurgical procedures have been developed, which are commonly based on extraction steps using electrolyte solutions containing a mixture of chloride and bromide (*Intec* process), ferric chloride (*Cuprex* process) or ferric sulfate (*Sepon* copper process). Chemical leaching processes may be combined with oxidation steps, or they are conducted under increased pressure and temperature. The *HydroCopper*^{®} method utilizes strong chloride solutions and copper(II) ions as oxidant (Haavanlamni, L., Hyvärinen O., Yllö, E., Weatherseed, M., HydroCopper® - A new method for treating copper concentrates, Extractive Metallurgy Operators' Conference 2005, Brisbane, Australia). King, J.A., Dreisinger, D.B. (Autoclaving of copper concentrates, Proc. Copper '95, Cobre '95. Montreal: CIM, 1995, pp. 511-534) describe a process comprising an autoclave treatment for destroying copper sulfide ores. Total pressure oxidation processes for the recovery of copper, cobalt and nickel from complex ores are also known for a long time (cf.: MccCormick W.R., Production of cobalt, nickel and copper at the Fredericktown Metal Refinery. Presented to the Mid America Minerals Conference of AIME 1958, St. Louis). Moreover, electrochemical extraction methods (e.g. the CALVANOX^{™} process) as described in WO 2005/118894 may be used for the recovery of copper from its ores and from concentrated copper ore. Due to passivation problems, the described methods may suffer from low yields and/or require sophisticated and expensive technical equipment, so that their economically feasibility is limited. This particularly applies if the copper containing material (ore or concentrated ore) contains high amounts of chalcopyrite, which is difficult to be dissolved in hydrometallurgical leaching processes due to a passivation of the mineral surface.

Recent approaches for separating copper from sulfide ores and concentrates apply bioleaching processes comprising the use of bacteria for the recovery of metals from the insoluble ore minerals. The bacteria convert the insoluble metal species into water soluble salts. In comparison with pyro- or hydrometallurgy, bioleaching processes are relatively simple to conduct, require low investment and operational costs, and are environmentally friendly in view of gaseous by-products and energy consumption.

Bacteria for use in bioleaching processes are categorized in three groups based on the growth temperature. According to the textbook Biology of Microorganisms (by Brock, Madigan, Martinko and Parker, 7th Ed., p.333), mesophilic bacteria have their growth optimum at 20-45 °C, thermophilic bacteria require temperatures of 45-80 °C and hyperthermophilic bacteria need a temperature above 85 °C.

Bioleaching may be conducted in two general ways - heap or tank leaching. Heap leaching is mostly conducted at low temperature, which limits the rate of sulfide mineral oxidation. Chalcopyrite cannot be processed at low temperatures at all. However, heating of the heap is in most cases difficult to be realized. Moreover, the restricted contact time between the leaching liquid and the ore material limits the yield of such leaching processes or increases the time needed to obtain a sufficient extraction result. Therefore, tank bioleaching is the method of choice when copper is to be separated from minerals as chalcopyrite, since the process parameters may be easily controlled and adapted to specific stages of the leaching process.

A number of bacteria have been investigated in the past. Sulfide ores and concentrated ores (other than chalcopyrite ores) may be oxidized by a mixed bacterial culture comprising predominantly *Thiobacillus ferrooxidans, Thiobacillus thiooxidans and Leptospirillum ferrooxidans,* which may operate at temperatures between 35 and 45 °C and at acidic pH (Dew, D.M., Miller, D.M., The BioNIC Process; Bioleaching of Mineral Sulfide Concentrates for Recovery of Nickel, In IBS Biomine '97, Sydney, p. M7.1.0-M7.1.9, or AU 689599). Other processes employ a mesophilic mixed bacterial culture comprising *Tiobacillus ferrooxidans*/*Leptospirillum ferrooxidans* (Brierley, C.L., Brans, R., 1994 - Selection of BacTech's Thermophilic Bio-Oxidation Process for Youanmi Mine, In Biomine 94, Conference Proceeding Perth Australia). However, these mesophilic bacterial cultures were unable to provide commercially acceptable recoveries from chalcopyrite in an acceptable leaching time.

One way of increasing the yield of bioleaching processes of chalcopyrite containing minerals may be the use of thermophilic bacteria. Bioleaching process using extremely thermophilic bacteria may result in high recovery of copper in a reasonable time, but such bacteria are very sensitive to changes in the process parameters as the content of solids and copper. In addition, extremely thermophilic bacteria require a temperature of more than 60 °C for growing, which is energy consuming and creates corrosive conditions.

A mixed bacterial culture comprising moderately thermophilic bacteria (leaching temperature 40-65 °C) comprising a mixture of *Sulfobacillus thermooxidans, Thiobacillus caldus* and *Thermobacillus ferrooxidans* is described in WO 01/18264. In this process 97 to 99 % of the copper are removed from chalcopyrite containing concentrates. However, the process needs between 20 and 36 days to obtain an acceptable recovery. The mixed bacterial culture described in WO 01/18264 is also utilized in a heap leaching process disclosed in US 2009/0061503. In addition, WO 02/066689 describes a heap leaching method employing a mixed stock bacterial culture, whereby no information regarding the yield is provided.

The bioleaching process described in WO 00/23629 employs moderately thermophilic bacteria, too. However, the copper yield does not exceed 93 % after one week of leaching.

Hence, there is a persistent need for fast, economically feasible and environmentally friendly processes for copper recovery from copper sulfide ores and concentrated copper ore predominantly containing chalcopyrite.

Surprisingly, it has been found that bioleaching using indigenous bacteria that are isolated from the ore to be processed are most effective in separating copper from these materials. Consequently, the current invention affords a novel tank bioleaching process using moderately thermophilic bacteria, which provides very high recoveries of copper in an acceptable period of time. The inoculant of the present invention contains indigenous bacteria isolated from the ore to be extracted. The bacteria in the inoculant are further adapted to the acidic process condition subsequently employed in the bioleaching process. In this way, the final inoculum is fully adapted to the extraction conditions prevailing in the bioleaching tank. The bioleaching process using the inoculant of the present invention allows the use of high contents of solid material during the bioleaching, while the temperature needed for growing and the efficacy of the employed bacteria may be easily controlled.

### BRIEF DESCRIPTION OF THE DRAWINGS

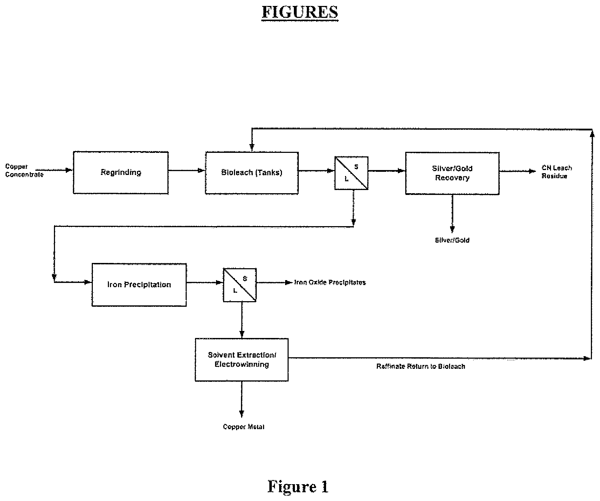

Figure 1 shows a generalized flowsheet of whole process comprising the bioleaching of copper concentrate using moderately thermophilic bacteria.

### DISCLOSURE OF THE INVENTION

In accordance with the present invention, an inoculant for microbial copper extraction from concentrated copper sulfide ores is provided, which is an aqueous suspension consisting of
a) a thermophilic, acidophilic bacterium isolated from the copper sulfide ore to be processed,
b) sulfuric acid,
c) nitrogen, phosphorus and potassium sources as nutrients,
d) dissolved oxygen, and
e) concentrated copper sulfide ore prepared from the copper sulfide ore to be processed.

The inoculant is characterized in that an indigenous bacterium is used, i.e. the bacterium is isolated from the ore to be processed.

The inoculant of the present invention is an aqueous suspension having a pH value of 3 or less, preferably 0.5-2, more preferred 0.9-1.1, and most preferred about 1.

Preferably, the inoculant has a dissolved oxygen content of at least 3 mg/l, preferably of at least 3.5 mg/l, and more preferred of at least 4 mg/l.

The temperature of the inoculant of the present invention may be adjusted to 40-65°C, preferably to 45-60 °C, and more preferred to 50-55 °C.

The content of the concentrated copper sulfide ore in the inoculant of the present invention may be 10 % (w/v) or less, preferably 3-8 % (w/v), more preferred 4-6 % (w/v), and most preferred about 5 % (w/v).

The cell density of the thermophilic, acidophilic bacterium, in the inoculant of the invention is usually 10⁸-10¹⁰ cells/ml, preferably about 10⁹ cells/ml.

In a preferred embodiment, the thermophilic, acidophilic bacterium is selected from *Thiobacillus* and *Sulfolobus.*

The inoculant of the present invention is suitable for extracting copper from concentrated copper sulfide ores in a tank leaching process.

The present disclosure also relates to a process for the separation of copper from concentrated copper sulfide ore comprising the steps:
a) preparation of an inoculant as defined above;
b) provision of an aqueous suspension of the concentrated copper sulfide ore;
c) start of the separation process by combining the suspension obtained in step (b) with the inoculant obtained in step (a) in a primary bioleaching tank to obtain a first slurry;
d) optionally continuation of the separation process after transfer of the first slurry into a secondary bioleaching tank;
e) optionally repeating step (d);
f) separation of solids from the slurry obtained in step (c), (d) or (e) to obtain a pregnant leach solution containing dissolved copper;
g) isolating the copper from the pregnant leach solution.

In the process of the present invention, the slurries obtained in steps (c), (d) and (e) have a content of concentrated copper sulfide ore of 18 % (w/v) or less, preferably of 5-15 % (w/v), more preferred of 8-12 % (w/v), and most preferred of about 10 % (w/v).

Preferably, the concentrated copper sulfide ore used in step (b) has a volume average particle size (Dᵥ50) of 30 µm or less, preferably of 20 µm or less, and more preferred of 15 µm or less.

The oxidation reduction potential (ORP) in steps (c), (d) and (e) may be adjusted to 350-480 mV, preferably to 365-465 mV, and more preferred to 380-450 mV.

In method steps (c), (d) and (e) the solution has a pH value of 3 or less, preferably of 0.5-2, more preferred of 0.9-1.1, and most preferred of about 1.

Preferably, during steps (c), (d) and (e) the solution has a dissolved oxygen content of at least 3 mg/l, preferably of at least 3.5 mg/l, and more preferred of at least 4 mg/l.

In method steps (c), (d) and (e) the temperature may be adjusted to 40-65 °C, preferably to 45-60 °C, and more preferred to 50-55 °C.

According to the process of the present invention, the concentrated copper sulfide ore has a copper content of 10-50 % by weight, preferably, 20-40 % by weight, and more preferred 25-35 % by weight.

In step (b) of the process of the present invention the same concentrated copper sulfide ore is used as in step (a) for the preparation of the inoculant.

### DESCRIPTION OF THE INVENTION

Each ore body has a slightly different stock of bacteria, which has been evolved under slightly different conditions such as temperature and chemistry of the ore. It has been found that by using such indigenous bacteria in a bioleaching process of sulfide copper ores or of a concentrated copper sulfide ore, copper may be extracted in high yields and in a short period of time.

Throughout the specification, an ore is considered a material that has been removed from the ground and does not receive any treatment to increase the metal content. A concentrated copper ore or copper concentrate is produced by passing an ore through a treatment process, generally gravity or flotation, in order to increase the concentration of the desired metals and decrease the volume of material, which is subsequently treated to recover those desired metals.

The indigenous bacteria culture used for the inoculant of the present invention is isolated from the ore to be processed. For this purpose, methods known in the art are applied (cf. e.g. WO 01/18264).

The inoculant according to the present invention is contains the isolated indigenous bacteria adapted to the process conditions employed in the subsequent bioleaching process. For this purpose, the bacteria are contacted with a nutrient medium (NPK medium) containing nitrogen, phosphorus and potassium as well as a content of the concentrated copper sulfide ore to be extracted. During growing of the inoculant, the pH is reduced to a value below 2, preferably to about 1, by the addition of sulfuric acid. The suspension is aerated and kept at a temperature of 40-65 °C, preferably of 45-60 °C, and more preferred of 50-55 °C. This generates an inoculant optimally adapted to the process conditions prevailing during the bioleaching of the concentrated copper sulfide ores.

For the initiation of the bioleaching of the concentrated copper ore, the inoculant is combined with a suspension of concentrated copper sulfide ore, whereby the concentrated copper sulfide ore is suspended in the same NPK medium as used for the inoculant. The pH, oxygen content and temperature are adjusted to the same values as employed for the preparation of the inoculant.

The inoculant contains bacteria, which optimally grow in a temperature range of 40-65 °C, preferably 45-60 °C, and more preferred 50-55 °C. Hence, they belong to the group of thermophilic bacteria, i.e. moderately thermophilic bacteria. They are capable of working at temperatures up to 65 °C and under acidic conditions, namely at pH values of between 0.9 and 2. The bacterial culture is dominated by bacteria belonging to the species of *Thiobacillus* and *Sulfolobus.*

The use of the inoculant results in a complete leaching of copper in an acceptable reaction time. The following examples illustrate the bioleaching process of the present invention.

### Example

### Preparation of the inoculant

The indigenous bacteria culture is firstly adapted to the ore material of interest. For this purpose, the stock bacteria are added to an NPK solution. Thereafter, concentrated copper sulfide ore is added to this suspension to reach a pulp with 5% solid density. The pH is adjusted to a value of about 1 by addition of sulfuric acid and by aeration the growth of bacteria is started. Aeration supports the mixing operation and provides enough oxygen and carbon dioxide, which accelerate the growth of bacteria. The content of oxygen is adjusted to a value of between 3 and 4 mg/l. Oxygen acts as electron acceptor during copper dissolution process.

After the natural bacteria adapted to the material of interest, the developed inoculant is combined with the sulfide ore or the concentrated sulfide ore, respectively, to be leached.

It has been found that the composition of the NPK medium strongly influences the growth of bacteria in the inoculant and the release of copper during the leaching. An efficient NPK medium may prepared dissolving in 1.0 L of water
- 0.8 g of Ammonium sulfate ((NH4)₂SO₄)
- 0.4 g of Potassium monophosphate (KH₂PO₄)
- 0.16 g of Magnesium sulphate (MgSO₄)
- 50 g of Ferrous sulfate pentahydrate (FeSO₄·7H₂O)

The pH of the solution is reduced to a values between 2.0 and 2.2 by the addition of 1-2 ml concentrated sulfuric acid (H₂SO₄) per liter of nutrient solution prepared.

The development of the inoculant usually takes three to four months.

Growing bacteria starts from laboratory scale and by upscaling reaches a volume of 2000 cubic meter solution containing each a bacteria concentration of about 10⁹ cell/ml. For this purpose, bacteria are grown in laboratory scale to provide a volume of 50 1 with a high concentration of bacteria. In the next step, bacteria growth is started in a tank with two cubic meter volume using the suspension from the small tank. Subsequently, step by step, the bacteria suspension is transferred into bigger tanks having volumes of 20, 200 or 2000 cubic meter. For all of these tanks the growing conditions are the same. For example, solution temperature will be kept around 50 to 55 °C using a heating/cooling system, the pH is adjusted to a value of about 1 by adding sulfuric acid and the content of dissolved oxygen should not be less than 4 mg/l. This is accomplished by the continuous aeration of the tanks.

The copper concentrate employed in the process should be grinded to a particle size of less than 15 µm. The increase of the specific surface supports the copper leaching by increasing the copper dissolution rate. The grinding of the copper concentrate is done by Vertimills.

### Bioleaching of concentrated copper sulfide ore

The bioleaching process is conducted in four tanks, which are connected with each other. Grinded copper concentrate, concentrated sulfuric acid, the prepared inoculant and diluent sulfuric acid solution are added to the first bioleaching reactor. The combination of these items will be done in such amounts that obtain a solution with 10 % solid density. The leaching conditions in biolaching tanks are the same as used for the preparation of the inoculant (pH value of about 1, oxygen content of about 4 mg/l, temperature of 50-55 °C). In addition, mechanical stirring is applied, which serves the effective mixing and suspension of the reactants. The copper concentrate in the four bioleaching tanks is agitated using agitators rotating at a circumferential velocity of 14.6, 14.6, 13.1 and 14.3 RPM (rounds per minute).

One of the problems of the microbiologically assisted dissolution of copper from its concentrated sulfide ores is the formation of passive layers such as *Jarosite,* sulfur and ferric components on concentrate's surface. Hence, in order to prevent the formation of theses layers, the redox potential in the bioleaching tanks is controlled to a value between 380 and 450 mV.

After adjusting the pH value of the suspension to about 1 and the desired temperature, the primary bioleaching process (process in the first tank) is started. In this step nearly 63 % of the copper from its concentrate is dissolved into the sulfuric acid solution, which provides a pregnant leach solution (PLS) having a copper content of 22 g/l after 3.5 days. Subsequently, the first PLS is transferred into the secondary tank, in which it has a retention time of about 1.2 days. During the process in the secondary tank, the copper concentration in the PLS increases to 29 g/l providing a copper recovery of 86 % at the end of this step.

In order to provide an almost complete extraction of copper from its concentrated sulfide ore, the developed process may comprise two further bioleaching steps. Following the same bioleaching method as employed in the first and second bioleaching tank, the content of dissolved copper in PLS will increase to 31.4 and 32.3 g/l, i.e. more than 94 and 97 % of copper will be dissolved in PLS after the third and fourth leaching step, respectively, which each may take up to 1.2 days.

Consequently, more than 97 % of the copper contained in the concentrated sulfide ore is dissolved within about 7 days in the bioleaching tanks. At the end of the bioleaching process, the produced pregnant leach solution (PLS) is fed to downstream process comprising, similarly to other bioleaching and chemical leaching processes of the art, at least one thickening step, at least one neutralization, a solvent extraction and finally the electro-winning in order to separate the solid residues from the bioleaching step and other impurities such as iron from the PLS, thereby providing a concentrated electrolyte. The latter is subjected to electro-winning, which finally provides a pure copper cathode.

### Downstream processes for purification and recovery of copper from PLS

The process mechanisms for downstream procedure are generally known. There are only some differences that will be explained hereinafter:
- Thickeners: The bioleaching outflow comprises an amount of solids, which must be separated from the PLS. For this purpose, the outflow is fed into two thickeners to separate PLS from solid particles. Thickeners over flow (PLS) is transferred to the next step and the solids (under flow) is transferred to a filter press.
- Filter press: Using a filter press, the solid humidity is decreased. The liquid pressed out is combined with the separated PLS.
- Neutralization: The PLS from filter presses and thickeners overflow are fed to neutralization tanks. In these tanks, by adding CaCO₃, the pH value of the solution is increased to about 2.7 leading to the precipitation of most of the iron in the form of Fe(OH)₃, which subsequently is separated from the PLS using thickeners. After thickening and filtering, the PLS is transferred to the PLS pond.

- Solvent extraction (SX): The solvent extraction serves the concentration and purification of PLS. In this part using a solvent and different diluents, the copper concentration in the PLS is increased to 30 g/l to 50 g/l Cu. The SX comprises three extraction and two stripping steps, which provide a concentrated electrolyte solution. To prevent the entry of organic particles into the concentrated electrolyte solution, which is subjected to electro-winning, a bank cyclone and an electrolyte filter is used to remove organic impurities from electrolyte.
- Electro-winning (EW): The concentrated electrolyte entering the EW part, is firstly fed into 30 scavenger cells. The copper concentration in the concentrated electrolyte entering the scavenger cells is about 50 g/l, while the content at the exit is about 47.3 g/l. The electrolyte is introduced subsequently to other cells. The formation of the desired copper plate on the surface of the cathode takes about 5 to 7 days.

The process of the present disclosure is efficient and fast. It completely dissolves copper from its concentrated sulfide ores within a period of only one week. Moreover, the process is environmentally friendly, requires low investment and operational costs, is simple and operates safe. Precious metals may be separated, too. Moreover, the process of the present disclosure can be applied to copper concentrates, which usually cannot easily be processed.

## Claims

1. An inoculant for microbial copper extraction from concentrated copper sulfide ores, which is an aqueous suspension consisting of:
a) a thermophilic, acidophilic bacterium isolated from the copper sulfide ore to be processed,
b) sulfuric acid,
c) nitrogen, phosphorus and potassium sources as nutrients,
d) dissolved oxygen, and
e) a concentrated copper sulfide ore prepared from the copper sulfide ore to be processed.

2. The inoculant according to claim 1, wherein the aqueous suspension has a pH of 3 or less, preferably 0.5-2, more preferred 0.9-1.1, and most preferred about 1.

3. The inoculant according to claim 1 or 2, wherein the dissolved oxygen content is at least 3 mg/l, preferably at least 3.5 mg/1, and more preferred at least 4 mg/l.

4. The inoculant according to any one of the preceding claims, wherein the temperature is adjusted to 40-65 °C, preferably to 45-60 °C, and more preferred to 50-55 °C.

5. The inoculants according to any one of the preceding claims, wherein the content of the concentrated copper sulfide ore is 10 % (w/v) or less, preferably 3-8 % (w/v), more preferred 4-6 % (w/v), and most preferred about 5 % (w/v).

6. The inoculant according to any one of the preceding claims, wherein the cell density of the thermophilic, acidophilic bacterium is 10⁸-10¹⁰ cells/ml, preferably about 10⁹ cells/ml.

7. The inoculant according to any one of the preceding claims, wherein the thermophilic, acidophilic bacterium is selected from *Thiobacillus* and *Sulfolobus.*

8. Use of an inoculant for extracting copper from concentrated copper sulfide ores, wherein the inoculant is an aqueous suspension consisting of:
a) a thermophilic, acidophilic bacterium isolated from the copper sulfide ore to be processed,
b) sulfuric acid,
c) nitrogen, phosphorus and potassium sources as nutrients,
d) dissolved oxygen, and
e) a concentrated copper sulfide ore prepared from the copper sulfide ore to be processed.

## Patentansprüche

1. Ein Inoculant für die mikrobielle Kupferextraktion aus konzentriertem Kupfersulfiderz, die als wässrige Suspension vorliegt, bestehend aus:
a) einem aus dem zu verarbeitenden Kupfersulfiderz isolierten, thermophilen, acidophilen Bakterium,
b) Schwefelsäure,
c) Stickstoff-, Phosphor- und Kaliumquellen als Nährstoffe,
d) gelöstem Sauerstoff und
e) einem aus dem zu verarbeitenden Kupfersulfiderz hergestellten, konzentrierten Kupfersulfiderz.

2. Inoculant gemäß Anspruch 1, wobei die wässrige Suspension einen pH-Wert von 3 oder weniger, vorzugsweise von 0,5-2, mehr bevorzugt von 0,9-1,1 und am meisten bevorzugt von ungefähr 1 aufweist.

3. Inoculant gemäß Anspruch 1 oder 2, wobei der Gehalt des gelösten Sauerstoffs wenigstens 3 mg/l, vorzugsweise wenigstens 3,5 mg/l und mehr bevorzugt wenigstens 4 mg/l beträgt.

4. Inoculant gemäß einem der vorstehenden Ansprüche, wobei die Temperatur auf 40-65 °C, vorzugsweise auf 45-60 °C und mehr bevorzugt auf 50-55 °C eingestellt wird.

5. Inoculant gemäß einem der vorstehenden Ansprüche, wobei der Gehalt an konzentriertem Kupfersulfiderz 10 % (Gew./Vol.) oder weniger, vorzugsweise 3-8 % (Gew./Vol.), mehr bevorzugt 4-6 % (Gew./Vol.) und am meisten bevorzugt ungefähr 5 % (Gew./Vol.) beträgt.

6. Inoculant gemäß einem der vorstehenden Ansprüche, wobei die Zelldichte der thermophilen, acidophilen Bakterien 10⁸-10¹⁰ Zellen/ml, vorzugsweise ungefähr 10⁹ Zellen/ml ist.

7. Inoculant gemäß einem der vorstehenden Ansprüche, wobei die thermophilen, acidophilen Bakterien aus *Thiobacillus* und *Sulfolobus* ausgewählt sind.

8. Verwendung eines Inoculants für die Extraktion von Kupfer aus konzentrierten Kupfersulfiderz, wobei das Inoculant eine wässrige Suspension ist, bestehend aus:
a) einem aus dem zu verarbeitenden Kupfersulfiderz isolierten, thermophilen, acidophilen Bakterium,
b) Schwefelsäure,
c) Stickstoff-, Phosphor- und Kaliumquellen als Nährstoffe,
d) gelöstem Sauerstoff und
e) einem aus dem zu verarbeitenden Kupfersulfiderz hergestellten, konzentrierten Kupfersulfiderz.

## Revendications

1. Inoculant pour une extraction microbienne de cuivre à partir de minerais concentrés de sulfure de cuivre, qui est une suspension aqueuse composée :
a) d'une bactérie thermophile et acidophile isolée du minerai de sulfure de cuivre à traiter,
b) d'acide sulfurique,
c) de sources d'azote, de phosphore et de potassium en tant que nutriments,
d) d'oxygène dissous, et
e) d'un minerai de sulfure de cuivre concentré préparé à partir du minerai de sulfure de cuivre à traiter.

2. Inoculant selon la revendication 1, dans lequel la suspension aqueuse a un pH de 3 ou moins, de préférence de 0,5 à 2, de manière plus préférée de 0,9 à 1,1, et de la manière la plus préférée d'environ 1.

3. Inoculant selon la revendication 1 ou 2, dans lequel la teneur en oxygène dissous est d'au moins 3 mg/l, de préférence d'au moins 3,5 mg/l, et de la manière la plus préférée d'au moins 4 mg/1.

4. Inoculant selon l'une quelconque des revendications précédentes, dans lequel la température est ajustée entre 40 et 65°C, de préférence entre 45 et 60°C, et de manière plus préférée entre 50 et 55°C.

5. Inoculant selon l'une quelconque des revendications précédentes, dans lequel la teneur du minerai de sulfure de cuivre concentré est de 10 % (p/v) ou moins, de préférence de 3 à 8 % (p/v), de manière plus préférée de 4 à 6 % (p/v). /v), et de la manière la plus préférée d'environ 5 % (p/v).

6. Inoculant selon l'une quelconque des revendications précédentes, dans lequel la densité cellulaire de la bactérie thermophile et acidophile est de 10⁸ à 10¹⁰ cellules/ml, de préférence d'environ 10⁹ cellules/ml.

7. Inoculant selon l'une quelconque des revendications précédentes, dans lequel la bactérie thermophile acidophile est choisie parmi *Thiobacillus* et *Sulfolobus.*

8. Utilisation d'un inoculant pour extraire du cuivre à partir de minerais concentrés de sulfure de cuivre, dans laquelle l'inoculant est une suspension aqueuse composée :
a) d'une bactérie thermophile et acidophile isolée du minerai de sulfure de cuivre à traiter,
b) d'acide sulfurique,
c) de sources d'azote, de phosphore et de potassium en tant que nutriments,
d) d'oxygène dissous, et
e) d'un minerai de sulfure de cuivre concentré préparé à partir du minerai de sulfure de cuivre à traiter.
